(19) Europäisches Patentamt
European Patent Office
Office européen des brevets

(11) Veröffentlichungsnummer: 0 393 423 A1

(12) # EUROPÄISCHE PATENTANMELDUNG

(21) Anmeldenummer: 90106458.4

(51) Int. Cl.5: C07D 253/06, A01N 43/64

(22) Anmeldetag: 04.04.90

(30) Priorität: 17.04.89 DE 3912508

(43) Veröffentlichungstag der Anmeldung: 24.10.90 Patentblatt 90/43

(84) Benannte Vertragsstaaten: BE CH DE FR GB IT LI NL

(71) Anmelder: BAYER AG
D-5090 Leverkusen 1 Bayerwerk(DE)

(72) Erfinder: Kranz, Eckart, Dr.
Am Acker 9
D-5600 Wuppertal 1(DE)
Erfinder: Santel, Hans-Joachim, Dr.
Grünstrasse 9a
D-5090 Leverkusen 1(DE)
Erfinder: Lürssen, Klaus, Dr.
August-Kierspel-Strasse 145
D-5060 Bergisch Gladbach 2(DE)
Erfinder: Schmidt, Robert R., Dr.
Im Waldwinkel 110
D-5060 Bergisch Gladbach 2(DE)

(54) **3,4-Disubstituierte 6-(1,1-Dichlor-2-methyl-2-propyl)-1,2,4,-triazin-5(4H)-one, Verfahren zu ihrer Herstellung und ihre Verwendung als Herbizide.**

(57) Die Erfindung betrifft neue 3,4-disubstituierte 6-(1,1-Dichlor-2-methyl-2-propyl)-1,2,4-triazin-5(4H)-one der Formel (I),

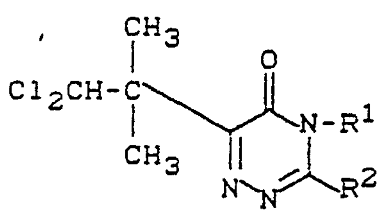

(I)

in welcher
$R^1$ für Amino oder Methyl steht und
$R^2$ für Alkylthio, Alkylamino oder Dialkylamino steht, wobei jedoch nicht gleichzeitig $R^1$ für Amino und $R^2$ für Methylthio stehen,
ein Verfahren zu ihrer Herstellung und ihre Verwendung als Herbizide.

EP 0 393 423 A1

# 3,4-Disubstituierte 6-(1,1-Dichlor-2-methyl-2-propyl)-1,2,4-triazin-5(4H)-one, Verfahren zu ihrer Herstellung und ihre Verwendung als Herbizide

Die vorliegende Erfindung betrifft neue 3,4-disubstituierte 6-(1,1-Dichlor-2-methyl-2-propyl)-1,2,4-triazin-5(4H)-one, ein Verfahren zu ihrer Herstellung und ihre Verwendung als Herbizide.

Es ist bereits bekannt, daß bestimmte 6-Dihalogen- bzw. Monohalogen-tert-butyl-1,2,4-triazin-5(4H)-on-Derivate, wie beispielsweise 4-Amino-6-(1,1-dichlor-2-methyl-2-propyl)-3-methylthio-1,2,4-triazin-5(4H)-on bzw. 3-Ethylmethylamino-4-methyl-6-(fluor-tert-butyl)-1,2,4-triazin-5(4H)-on, herbizide Eigenschaften besitzen (vgl. EP-A 0 130 517 bzw. EP-A 0 049 416). Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Es wurden die neuen 3,4-disubstituierten 6-(1,1-Dichlor-2-methyl-2-propyl)-1,2,4-triazin-5(4H)-one der Formel (I)

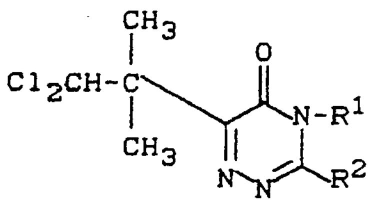

(I)

in welcher

$R^1$ für Amino oder Methyl steht und

$R^2$ für Alkylthio, Alkylamino oder Dialkylamino steht, wobei jedoch nicht gleichzeitig $R^1$ für Amino und $R^2$ für Methylthio stehen,

gefunden.

Weiterhin wurde gefunden, daß man die neuen 3,4-disubstituierten 6-(1,1-Dichlor-2-methyl-2-propyl)-1,2,4-triazin-5(4H)-one der Formel (I) erhält, wenn man in einer ersten Stufe 6-(1,1-Dichlor-2-methyl-2-propyl)-3-mercapto-1,2,4-triazin-5(4H)-on-Derivate der Formel (II)

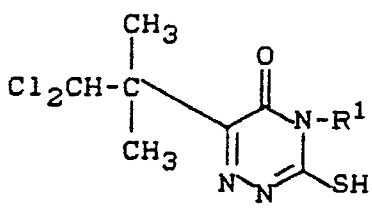

(II)

in welcher

$R^1$ die oben angegebene Bedeutung hat,

in an sich bekannter Weise in alkalischer Lösung mittels Alkylhalogenid, vorzugsweise Alkyliodid oder -bromid, zu 3-Alkylthio-6-(1,1-dichlor-2-methyl-2-propyl)-1,2,4-triazin-5(4H)-on-Derivaten der Formel (Ia)

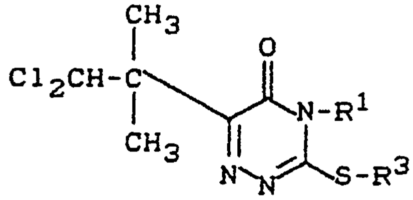

(Ia)

in welcher

$R^3$ für Alkyl, vorzugsweise mit 1 bis 4 Kohlenstoffatomen, steht und

$R^1$ die oben angegebene Bedeutung hat,

alkyliert und die Verbindungen der Formel (Ia) gegebenenfalls in einer zweiten Stufe mit Aminen der Formel

(III)

$HNR^4R^5$ (III)

in welcher

$R^4$ für Wasserstoff oder Alkyl, vorzugsweise mit 1 bis 4 Kohlenstoffatomen, steht und

$R^5$ für Alkyl, vorzugsweise mit 1 bis 4 Kohlenstoffatomen, steht,

gegebenenfalls in Gegenwart eines Verdünnungsmittels und gegebenenfalls in Gegenwart einer niederen aliphatischen Carbonsäure umsetzt.

Schließlich wurde gefunden, daß die neuen 3,4-disubstituierten 6-(1,1-Dichlor-2-methyl-2-propyl)-1,2,4-triazin-5(4H)-one der Formel (I) herbizide, insbesondere selektiv-herbizide Eigenschaften besitzen.

Überraschenderweise zeigen die erfindungsgemäßen 3,4-disubstituierten 6-(1,1-Dichlor-2-methyl-2-propyl)-1,2,4-triazin-5(4H)-one der Formel (I) neben einer besseren herbiziden Wirksamkeit gegenüber wichtigen Problemunkräutern gleichzeitig eine deutlich verbesserte Verträglichkeit gegenüber wichtigen Kulturpflanzen, wie insbesondere Weizen, beispielsweise im Vergleich zu 3-Ethylmethylamino-4-methyl-6-(fluor-tert-butyl)-1,2,4-triazin-5(4H)-on, welches chemisch und wirkungsmäßig eine naheliegende Verbindung ist.

Die erfindungsgemäßen 3,4-disubstituierten 6-(1,1-Dichlor-2-methyl-2-propyl)-1,2,4-triazin-5(4H)-one sind durch die Formel (I) allgemein definiert. In dieser Formel stehen vorzugsweise

$R^1$ für Amino oder Methyl und

$R^2$ für geradkettiges oder verzweigtes Alkylthio mit 1 bis 4 Kohlenstoffatomen, für geradkettiges oder verzweigtes Alkylamino mit 1 bis 4 Kohlenstoffatomen sowie für geradkettiges oder verzweigtes Dialkylamino mit 1 bis 4 Kohlenstoffatomen in jedem Alkylteil, wobei jedoch nicht gleichzeitig $R^1$ für Amino und $R^2$ für Methylthio stehen.

Besonders bevorzugt sind diejenigen Verbindungen der Formel (I), in denen

$R^1$ für Amino oder Methyl steht und

$R^2$ für Methyl-, Ethyl- oder Propylthio; für Methyl-, Ethyl- Propyl- oder Butylamino; sowie für Dimethyl-, Diethyl- oder Ethylmethylamino steht, wobei jedoch nicht gleichzeitig $R^1$ für Amino und $R^2$ für Methylthio stehen.

Verwendet man beispielsweise 6-(1,1-Dichlor-2-methyl-2-propyl)-3-mercapto-4-methyl-1,2,4-triazin-5-(4H)-on und Methyliodid als Ausgangsstoffe, so läßt sich der Reaktionsablauf der ersten Stufe des erfindungsgemäßen Verfahrens durch das folgende Formelschema wiedergeben:

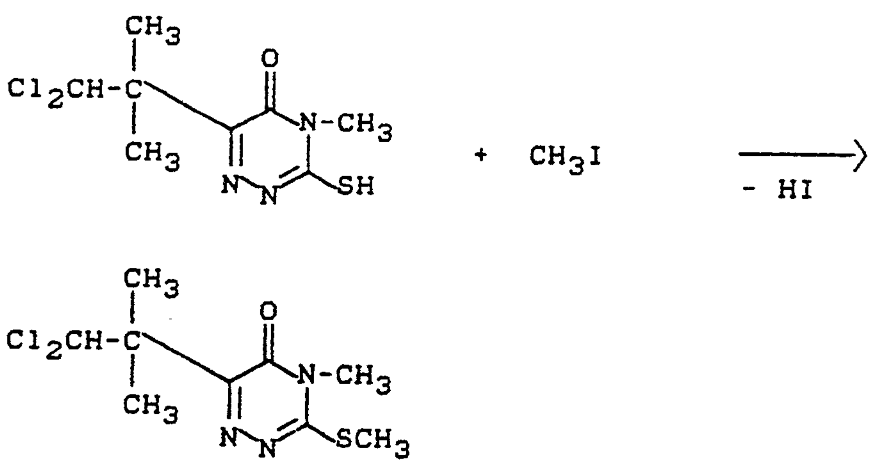


Verwendet man beispielsweise 6-(1,1-Dichlor-2-methyl-2-propyl)-4-methyl-3-methylthio-1,2,4-triazin-5-(4H)-on und Methylamin als Ausgangsstoffe, so läßt sich der Reaktionsablauf der zweiten Stufe des erfindungsgemäßen Verfahrens durch das folgende Formelschema wiedergeben:

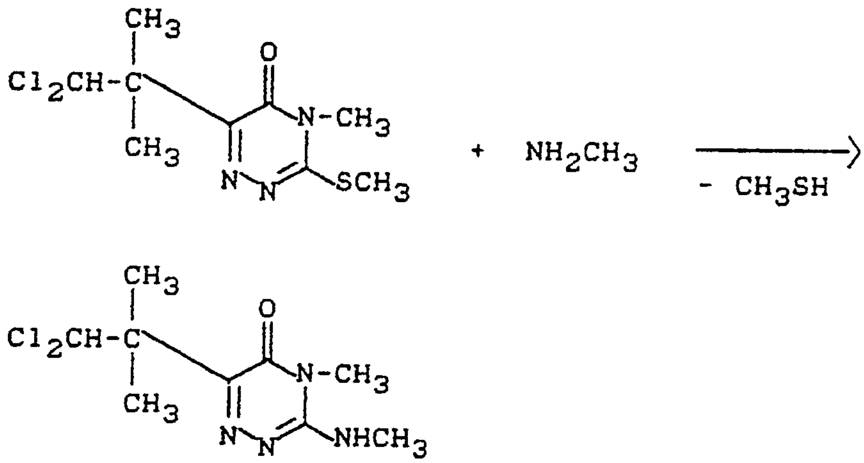

Die zur Durchführung des erfindungsgemäßen Verfahrens als Ausgangsstoffe benötigten 6-(1,1-Dichlor-2-methyl-2-propyl)-3-mercapto-1,2,4-triazin-5(4H)-on-Derivate sind durch die Formel (II) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden.

Die Ausgangsstoffe der Formel (II) werden in bekannter Art und Weise erhalten, indem man 3,3-Dichlor-2,2-dimethyl-propionsäurechlorid und Trimethylsilylcyanid umsetzt; das erhaltene 3,3-Dichlor-2,2-dimethyl-propionylcyanid gegebenenfalls in Gegenwart einer flüssigen Carbonsäure, wie z.B. Essigsäure, als Lösungsmittel mit einer anorganischen Säure, wie z.B. Bromwasserstoff umsetzt; und schließlich das erhaltene 3-Dichlormethyl-3,3-dimethyl-brenztraubensäureamid der Formel (IV)

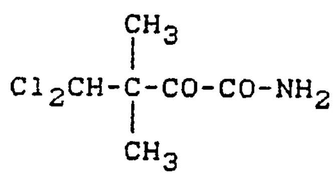
(IV)

entweder in der anfallenden Lösung oder nach Zwischenisolierung, gegebenenfalls nach vorheriger Verseifung zu der freien Säure, mit Verbindungen der Formel (V)

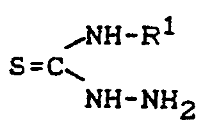
(V)

in welcher

$R^1$ die oben angegebene Bedeutung hat,

in wässriger bzw. in wässrig-saurer Lösung, gegebenenfalls in Gegenwart eines organischen Verdünnungsmittels, wie z.B. Dimethylformamid, zu den 6-(1,1-Dichlor-2-methyl-2-propyl)-3-mercapto-1 ,2,4-triazin-5(4H)-on-Derivaten der Formel (II) cyclisiert (vgl. hierzu z.B. EP-A 0 049 416 und EP 0 130 517 sowie die Herstellungsbeispiele).

Die Amine der Formel (III) und die Verbindungen der Formel (V) sind allgemein bekannte Verbindungen der organischen Chemie.

Die erste Stufe des erfindungsgemäßen Verfahrens wird in Gegenwart einer Base durchgeführt. Hierbei werden vorzugsweise Alkalihydroxide, wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung der ersten Stufe des erfindungsgemäßen Verfahrens in einem größeren Bereich variiert werden. Im allgemeinen arbeitet man zwischen 0° und 100° C, vorzugsweise zwischen 0° und 50° C.

Bei der Durchführung der ersten Stufe des erfindungsgemäßen Verfahrens setzt man auf 1 Mol an Verbindung der Formel (II) vorzugsweise 1 bis 1,5 Mol Alkylierungs mittel ein. Die Isolierung der

Zwischenprodukte bzw. Endprodukte der Formel (Ia) erfolgt in üblicher Weise.

Bei der zweiten Stufe des erfindungsgemäßen Verfahrens kommen als Verdünnungsmittel alle inerten organischen Lösungsmittel infrage. Hierzu gehören Kohlenwasserstoffe, wie Toluol, Xylol; chlorierte aromatische Kohlenwasserstoffe, wie Chlorbenzol, 1,2-Dichlorbenzol, 1,2,4-Trichlorbenzol; Ether, wie Tetrahydrofuran, Dioxan; Alkohole, wie Methanol, Ethanol, Propanol, Isopropanol; Amide, wie N,N-Dimethylformamid, Tetramethylharnstoff oder Sulfoxide, wie Dimethylsulfoxid. Vorzugsweise wird für die Umsetzung Isopropanol verwendet.

Die Reaktionstemperaturen können bei der zweiten Stufe des erfindungsgemäßen Verfahrens in einem größeren Bereich variiert werden. Im allgemeinen arbeitet man bei Temperaturen zwischen 20 °C und 170 °C, vorzugsweise bei Temperaturen zwischen 60 °C und 90 °C.

Eine besonders vorteilhafte Ausführungsform der zweiten Stufe des erfindungsgemäßen Verfahrens besteht darin, daß man in Gegenwart mindestens der äquimolaren Menge einer niederen aliphatischen Carbonsäure arbeitet. Vorzugsweise wird hierfür Essigsäure verwendet. Dieses Verfahren gestattet es mit relativ geringem Amin-Überschuß auszukommen. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfonsäure.

Bei der Durchführung der zweiten Stufe des erfindungsgemäßen Verfahrens setzt man auf 1 Mol des 3-Alkylthiotriazinons der Formel (Ia) zweckmäßigerweise 1 bis 3 Mol einer niederen aliphatischen Carbonsäure und gegebenenfalls 0,01 bis 0,05 Mol einer organischen Sulfonsäure sowie 1 bis 7 Mol Amin der Formel (III) ein, erhitzt bis zum Ende der Mercaptan-Abspaltung und arbeitet anschließend in üblicher Art und Weise auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der an gewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung mono- und dikotyler Unkräuter im Weizen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen des Wirkstoffs mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-BP); 3-Isopropyl-2,1,3-benzothiadiazin-4-on-2,2-dioxid (BENTAZON); Methyl-5-(2,4-dichlorphenoxy)-2-nitrobenzoat (BIFENOX): 3,5-Dibrom-4-hydroxy-benzonitril (BROMOXYNIL); N-(Butoxymethyl)-2-chlor-N-(2,6-diethylphenyl)-acetamid (BUTACHLOR); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); α-Chlor-2′,6′-diethyl-N-(2-propoxyethyl)-acetanilid (PRETILACHLOR); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und N,N-Diisopropyl-5-(2,3,3-trichlorallyl)-thiolcarbamat (TRIALLATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe gehen aus den nachfolgenden Beispielen hervor.

Herstellungsbeispiele

Beispiel 1

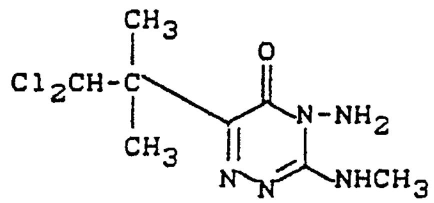

14,2 g (0,05 Mol) 4-Amino-6-(1,1-dichlor-2-methyl-2-propyl)-3-methylthio-1,2,4-triazin-5(4H)-on werden bei 5 bis 10° C zu einer Lösung aus 150 ml Isopropanol, 12 g (0,2 Mol) Eisessig und 13,4 g (0,4 Mol) Methylamin gegeben. Man läßt auf Raumtemperatur erwärmen und erhitzt anschließend 14 Stunden unter Rückfluß. Danach wird das Reaktionsgemisch eingeengt und der ölige Rückstand in Wasser eingerührt. Man extrahiert mit Methylenchlorid, wäscht mit Wasser, trocknet über Natriumsulfat und engt ein Der Rückstand wird über eine Kieselgel-Säule (Fließmittel: Methylenchlorid, Essigester) gereinigt. Man erhält 8,5 g (63 % der Theorie) 4-Amino-6-(1,1-dichlor-2-methyl-2-propyl)-3-methylamino-1,2,4-triazin5(4H)-on vom Schmelzpunkt 192-193° C.

Herstellung des Ausgangsproduktes

(a)

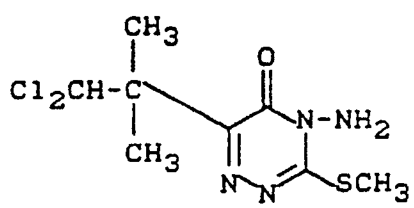

43,5 g (0,16 Mol) 4-Amino-6-(1,1-dichlor-2-methyl-2-propyl)-3-mercapto-1,2,4-triazin-5(4H)-on werden in 178 ml 1N Natronlauge gelöst und vom unlöslichen Rückstand abfiltriert. Das Filtrat wird bei Raumtemperatur mit 25,3 g (0,18 Mol) Methyliodid versetzt und über Nacht gerührt. Der ausgefallene Feststoff wird abgesaugt, mit Wasser gewaschen, im Vakuum getrocknet, dann mit Methylenchlorid kalt verrührt und abgesaugt.

Man erhält 21,1 g (46 % der Theorie) 4-Amino-6-(1,1-dichlor-2-methyl-2-propyl)-3-methylthio-1,2,4-triazin-5(4H)-on vom Schmelzpunkt 173-175° C.

(b)

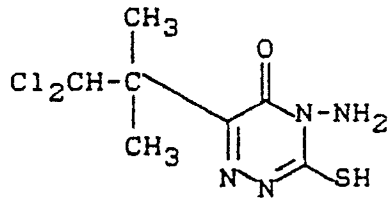

49,5 g (0,25 Mol) 3-Dichlormethyl-3,3-dimethyl-brenztraubensäureamid werden bei Raumtemperatur zu einer filtrierten Lösung von 29,3 g (0,275 Mol) Thiocarbohydrazid in 275 ml 1N Salzsäure gegeben. Man rührt das Reaktionsgemisch 8 Stunden bei 95 °C. Der ausgefallene Niederschlag wird abgesaugt, mit Wasser gewaschen und bei 50 °C im Vakuum getrocknet.

Man erhält 34,7 g (51,6 % der Theorie) 4-Amino-6-(1,1-dichlor-2-methyl-2-propyl)-3-mercapto-1,2,4-triazin-5(4H)-on vom Schmelzpunkt 198-200 °C.

(c)

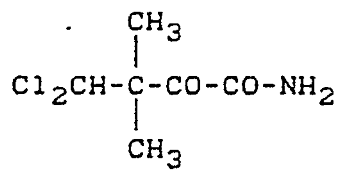

47,0 g (0,25 Mol) 3,3-Dichlor-2,2-dimethylpropionylcyanid werden bei Raumtemperatur zu 200 g Bromwasserstoff in Eisessig gegeben. Man läßt 1 Stunde bei Raumtemperatur nachrühren und versetzt unter Kühlung mit 4,5 g (0,25 Mol) Wasser, so daß die Innentemperatur 25 °C nicht übersteigt. Man läßt eine weitere Stunde nachrühren und gibt das Reaktionsgemisch auf 250 g Eiswasser. Der ausgefallene weiße Niederschlag wird abgesaugt, mit Wasser neutral gewaschen und bei Raumtemperatur im Vakuum getrocknet.

Man erhält 45,3 g (91,5 % der Theorie) 3-Dichlormethyl-3,3-dimethyl-brenztraubensäureamid vom Schmelzpunkt 97-99 °C.

(d)

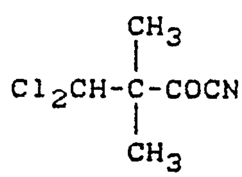

140,0 g (0,74 Mol) 3,3-Dichlor-2,2-dimethyl-propion säurechlorid werden auf 110-120 °C erwärmt. Bei dieser Temperatur tropft man innerhalb von 60 Minuten 74,0 g (0,75 Mol) Trimethylsilylcyanid zu. Gleichzeitig wird das entstehende Trimethylsilylchlorid destillativ aus der Reaktionsmischung entfernt. Man läßt 30 Minuten bei 150 °C nachrühren und destilliert das Reaktionsprodukt über eine 60 cm Spiegelvigreuxkolonne mit Rektifikationsaufsatz (Rücklauf 6:1).

Man erhält 175,2 g (72,0 % der Theorie) 3,3-Dichlor-2,2-dimethyl-propionylcyanid vom Siedepunkt 82 °C bei 25 mbar

(e)

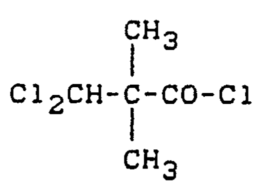

297,5 g (2,5 Mol) Thionylchlorid werden bei Raumtemperatur langsam zu einer Lösung aus 273,6 g (1,3 Mol) 3,3-Dichlor-2,2-dimethyl-propionsäure in 250 ml Chloroform getropft. Die Reaktionslösung wird anschließend ca. 8 Stunden bei Rückfluß nachgerüht, bis die Gasentwicklung beendet ist. Die Reaktionsmischung wird fraktioniert.

Man erhält 210,3 g (85,4 % der Theorie) 3,3-Dichlor-2,2-dimethyl-propionsäurechlorid vom Siedepunkt 71-73 °C bei 22 mbar.

Beispiel 2

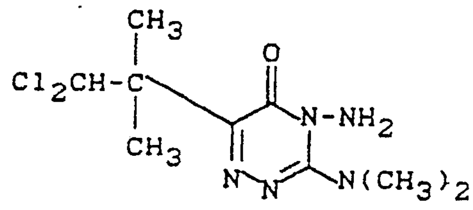

14,2 g (0,05 Mol) 4-Amino-6-(1,1-dichlor-2-methyl-2-propyl)-3-methylthio-1,2,4-triazin-5(4H)-on (vgl. Bsp. 1a) werden bei 5-10 °C zu einer Lösung aus 150 ml Isopropanol, 12 g (0,2 Mol) Eisessig und 18 g (0,4 Mol) Dimethylamin gegeben. Man läßt auf Raumtemperatur erwärmen und erhitzt anschließend 14 Stunden unter Rückfluß. Danach wird das Isopropanol im Vakuum abdestilliert und der Rückstand wird in Methylenchlorid aufgenommen; diese Lösung wird mit Wasser gewaschen, über Natriumsulfat getrocknet und eingeengt. Der ölige Rückstand wird über eine Kieselgel-Säule gereinigt.

Man erhält 10,0 g (71,4 % der Theorie) 4-Amino-6-(1,1-dichlor-2-methyl-2-propyl)-3-dimethylamino-1,2,4-triazin-5(4H)-on vom Schmelzpunkt 140-141 °C.

Beispiel 3

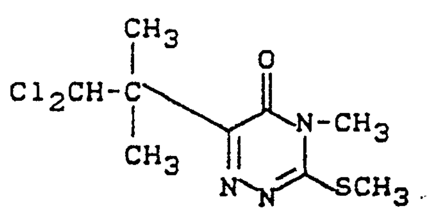

80,4 g (0,3 Mol) 6-(1,1-Dichlor-2-methyl-2-propyl)-3-mercapto-4-methyl-1,2,4-triazin-5(4H)-on werden in 300 ml 1N Natronlauge gelöst. Eventuelle unlösliche Verunreinigungen werden abfiltriert. Zu der klaren Lösung tropft man bei Raumtemperatur 51,1 g (0,36 Mol) Methyliodid und läßt über Nacht bei dieser Temperatur nachrühren. Der entstehende Feststoff wird abgesaugt, mit Wasser gewaschen, im Vakuum getrocknet und aus Essigester umkristallisiert.

Man erhält 47 g (55,5 % der Theorie) 6-(1,1-Dichlor-2-methyl-2-propyl)-4-methyl-3-methylthio-1,2,4-triazin-5-(4H)-on vom Schmelzpunkt 158 °C.

Herstellung des Ausgangsproduktes

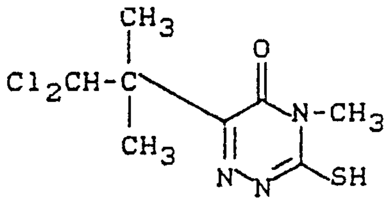

147 g (0,8 Mol) 3,3-Dichlor-2,2-dimethylpropionylcyanid werden bei Raumtemperatur zu 800 g Bromwasserstoff in Eisessig gegeben. Man läßt 30 Minuten bei Raumtemperatur nachrühren und gibt dann 155 g (1,5 Mol) Methylthiosemicarbazid in 1,5 l 1N Salzsäure zu, wobei die Innentemperatur durch Kühlung auf ca. 15° C gehalten wird. Man läßt über Nacht bei 95° C nachrühren. Nach dem Abkühlen auf Raumtemperatur wird der ausgefallene Niederschlag abgesaugt und getrocknet.

Man erhält 58,5 g (27,3 % der Theorie) 6-(1,1-Dichlor-2-methyl-2-propyl)-3-mercapto-4-methyl-1,2,4-triazin-5(4H)-on vom Schmelzpunkt 168-72° C.

Beispiel 4

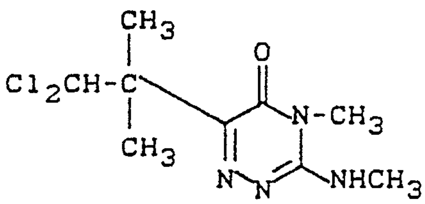

13,0 g 6-(1,1-Dichlor-2-methyl-2-propyl)-4methyl-3-methylthio-1,2,4-triazin-5(4H)-on (Bsp. 3) werden bei 0° bis 5° C zu einer Lösung aus 50 ml Isopropanol, 5,5 g (0,092 Mol) Eisessig und 4,2 g (0,14 Mol) Methylamin gegeben. Man läßt auf Raumtemperatur erwärmen und erhitzt anschließend ca. 20 Stunden unter Rückfluß. Danach wird das Reaktionsgemisch eingeengt und der Rückstand in Wasser eingerührt. Man extrahiert mit Methylenchlorid, wäscht mit Wasser, trocknet über Natriumsulfat und engt ein. Der Rückstand wird in n-Hexan kalt verrührt und abgesaugt.

Man erhält 11,3 g (93 % der Theorie) 6-(1,1-Dichlor-2-methyl-2-propyl)-4-methyl-3-methylamino-1,2,4-triazin-5(4H)-on vom Schmelzpunkt 172-74° C.

In analoger Weise und entsprechend dem erfindungsgemäßen Verfahren werden die in der nachfolgenden Tabelle aufgeführten Verbindungen der Formel (I) erhalten:

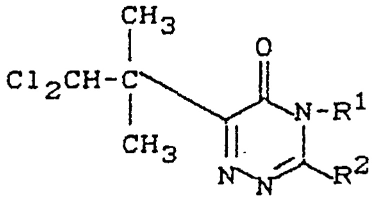

(I)

| Bsp. Nr. | $R^1$ | $R^2$ | Schmelzpunkt (°C) |
|---|---|---|---|
| 5 | $-NH_2$ | 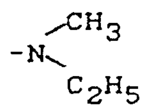 | 109-110 |
| 6 | $-CH_3$ | $-N(CH_3)_2$ | 82-84 |
| 7 | $-CH_3$ | 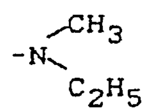 | 54-56 |
| 8 | $-CH_3$ | $-SC_2H_5$ | 135-137 |

## Anwendungsbeispiele

In den folgenden Anwendungsbeispielen wird die nachstehend aufgeführte Verbindung als Vergleichssubstanz eingesetzt:

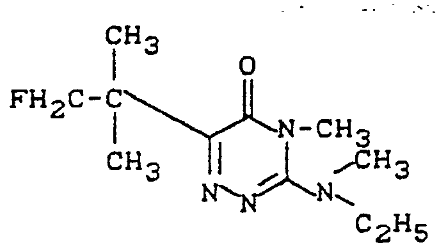

(A)

(bekannt aus EP-A-0 049 416)

## Beispiel A

Pre-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit

der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen: 1, 2, 4, 6 und 7.

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen: 1, 2, 4, 6 und 7.

**Ansprüche**

1. 3,4-Disubstituierte 6-(1,1-Dichlor-2-methyl-2-propyl)-1,2,4-triazin-5(4H)-one der Formel (I)

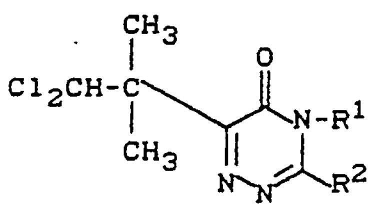

(I)

in welcher

$R^1$ für Amino oder Methyl steht und

$R^2$ für Alkylthio, Alkylamino oder Dialkylamino steht, wobei jedoch nicht gleichzeitig $R^1$ für Amino und $R^2$ für Methylthio stehen.

2. Triazinon-Derivate der Formel (I) gemäß Anspruch 1, dadurch gekennzeichnet, daß darin

$R^1$ für Amino oder Methyl steht und

$R^2$ für geradkettiges oder verzweigtes Alkylthio mit 1 bis 4 Kohlenstoffatomen, für geradkettiges oder verzweigtes Alkylamino mit 1 bis 4 Kohlenstoffatomen sowie für geradkettiges oder verzweigtes Dialkylamino mit 1 bis 4 Kohlenstoffatomen in jedem Alkylteil steht, wobei jedoch nicht gleichzeitig $R^1$ für Amino und

$R^2$ für Methylthio stehen.

3. Triazinon-Derivate der Formel (I) gemäß Anspruch 1, dadurch gekennzeichnet, daß darin
$R^1$ für Amino oder Methyl steht und
$R^2$ für Methyl-, Ethyl- oder Propylthio; für Methyl-, Ethyl-, Propyl- oder Butylamino; sowie für Dimethyl-, Diethyl- oder Ethylmethylamino steht, wobei jedoch nicht gleichzeitig $R^1$ für Amino und $R^2$ für Methylthio stehen.

4. Verfahren zur Herstellung von 3,4-disubstituierten 6-(1,1-Dichlor-2-methyl-2-propyl)-1,2,4-triazin-5-(4H)-onen der Formel (I) gemäß Anspruch 1, dadurch gekennzeichnet, daß man in einer ersten Stufe 6-(1,1-Dichlor-2-methyl-2-propyl)-3-mercapto-1,2,4-triazin-5(4H)-on-Derivate der Formel (II)

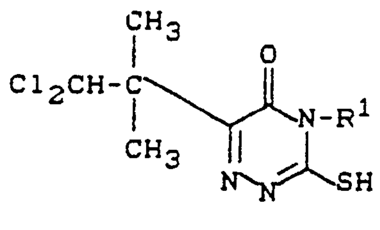

(II)

in welcher
$R^1$ die in Anspruch 1 angegebene Bedeutung hat,
in alkalischer Lösung mittels Alkylhalogenid, vorzugsweise Alkyliodid oder -bromid, zu 3-Alkylthio-6-(1,1-dichlor-2-methyl-2-propyl)-1,2,4-triazin-5(4H)-on-Derivaten der Formel (Ia)

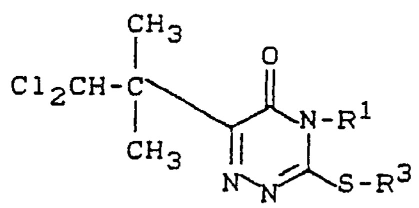

(Ia)

in welcher
$R^3$ für Alkyl, vorzugsweise mit 1 bis 4 Kohlenstoffatomen, steht und
$R^1$ die oben angegebene Bedeutung hat, alkyliert und die Verbindungen der Formel (Ia) gegebenenfalls in einer zweiten Stufe mit Aminen der Formel (III)
$HNR^4R^5$ (III)
in welcher
$R^4$ für Wasserstoff oder Alkyl, vorzugsweise mit 1 bis 4 Kohlenstoffatomen, steht und
$R^5$ für Alkyl, vorzugsweise mit 1 bis 4 Kohlenstoffatomen, steht,
gegebenenfalls in Gegenwart eines Verdünnungsmittels und gegebenenfalls in Gegenwart einer niederen aliphatischen Carbonsäure umsetzt.

5. Herbizide Mittel, gekennzeichnet durch einen Gehalt an mindestens einem Triazinon-Derivat der Formel (I) gemäß Anspruch 1.

6. Verfahren zur Bekämpfung von Unkraut, dadurch gekennzeichnet, daß man Triazinon-Derivate der Formel (I) gemäß Anspruch 1 auf die Unkräuter oder ihren Lebensraum einwirken läßt.

7. Verwendung von Triazinon-Derivaten der Formel (I) gemäß Anspruch 1 zur Bekämpfung von unerwünschtem Pflanzenwachstum.

8. Verfahren zur Herstellung von herbiziden Mitteln, dadurch gekennzeichnet, daß man Triazinon-Derivate der Formel (I) gemäß Anspruch 1 Streckmitteln und/oder oberflächenaktiven Stoffen vermischt.

Europäisches Patentamt

# EUROPÄISCHER RECHERCHENBERICHT

Nummer der Anmeldung

EP 90106458.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2 - 0 049 416 (BAYER) * Ansprüche 1,7-10 * -- | 1,5-8 | C 07 D 253/06<br>A 01 N 43/64 |
| D,A | EP - A2 - 0 130 517 (BAYER) * Ansprüche 1,6-8; Beispiel 10 * -- | 1,5-8 | |
| A | DE - A1 - 3 135 413 (BAYER) * Ansprüche 1,8-10; Beispiel 1 * -- | 1,5-8 | |
| A | DE - A1 - 3 339 859 (BAYER) * Beispiel 15; Anspruch 1 * ---- | 1,4 | |

RECHERCHIERTE SACHGEBIETE (Int Cl 5)

C 07 D 253/00

Der vorliegende Recherchenbericht wurde für alle Patentansprüche erstellt.

| Recherchenort | Abschlußdatum der Recherche | Prüfer |
|---|---|---|
| WIEN | 06-06-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82